# EUROPEAN PATENT SPECIFICATION

(11) **EP 0 567 424 B1**
(45) Date of publication and mention of the grant of the patent: **01.07.1998**
(21) Application number: 93500051.3
(22) Date of filing: 23.04.1993
(51) Int. Cl.: A61F 2/44, A61B 17/60

(54) **Vertebral prosthesis for the substitution of a vertebra in malignant tumour surgery**
Wirbelprothese zum Ersetzen eines Wirbels in der Chirurgie maligner Tumore
Prothèse vertébrale de remplacement d'une vertèbre dans la chirurgie d'une tumeur maligne

(30) Priority: 24.04.1992 ES 9200869
(43) Date of publication of application: 27.10.1993
(73) Proprietor: Barbera Alacreu, José Vicente, 46010 Valencia (ES)
(72) Inventor: Barbera Alacreu, José Vicente, E-46010 Valencia (ES)
(74) Representative: Blumbach, Kramer & Partner GbR

(56) References cited:
- EP-A- 0 179 695
- EP-A- 0 188 954
- DE-A- 3 023 942
- DE-U- 9 107 494
- DE-U- 9 112 176
- FR-A- 2 636 227
- GB-A- 2 083 754
- US-A- 4 553 273
- US-A- 4 932 975

## Description

The present invention relates to a prosthesis assembly for the substitution of a vertebra in malignant tumour surgery, providing, for the purpose to which it is directed, various advantages which will be listed hereinafter, apart from others inherent in the organization and constitution thereof.

It is known that the most appropriate technique and the one currently most used by spinal column surgeons in the treatment of malignant vertebral tumours is anterolateral decompression, by resecting the affected vertebra. Therewith, further to freeing the spinal cord, the volume of the tumour is significantly reduced, making it more accessible to post-operative radiotherapy and/or chemotherapy.

But resection of the vertebra of necessity causes a maximum instability which must be corrected during the operation itself, although at a later stage, in a second session it may be desirable to complete the stabilization with posterior instrumentation.

Stabilization involves a reconstruction of the anatomical continuity of the column, making it capable of supporting the load and allowing the patient freedom of movement, without complications such as pain, deformity or new nerve compression deriving therefrom.

The methods currently used in surgery for vertebral reconstruction are:
1) bone graft, associated with anterior instrumentation;
2) methyl methacrylate, associated with anterior instrumentation;
3) vertebral prosthesis.

In the particular case of vertebral prostheses, which is the field of the present invention, experimental prostheses of very complex design are currently known for benign tumours of the cervical, thoracic and lumbar portions of the spinal column, but have not been used on human patients.

The document GB-A-2,083,754 discloses a spinal fixator in the form of a turnbuckle the end sections of which terminate as circular flat plates formed with upwardly and downwardly protruding spikes for engaging in the bone of the adjacent vertebrae above and below a broken vertebra. The platform members have a diameter which is similar to the diameter of the fixator body. GB-A-2 083 754 further discloses a threaded bolt to be engaged with the fixator body and a plate including a central vertical slot for receiving said threaded bolt.

In the belief that fitting of a prosthesis should, theoretically, not suffer from any of the drawbacks of the current techniques, the present inventors have designed a vertebral prosthesis for use in the surgery of malign tumours of the dorsal and lumbar portions of the spinal column.

The invention is directed to a prosthesis assembly (2) for replacing a vertebra in a spine, said assembly comprising
- a center body member (4) in the form of a hollow tube having an internal thread (7) and diametral threaded holes (17);
- a pair of platform members (5, 6) each having a plate member (8) and a cylinder part (10), said cylinder part (10) having an outer thread (11) to be inserted into one end of said hollow tube of the center body member (4);
- a threaded bolt (16) to be engaged with one of the diametral threaded holes (17) of the center body member (4); and
- a plate (3) including a center hole (15) for receiving the threaded bolt (16) and additional holes (13) for receiving spongiosa screws (14) to be screwed into vertebrae adjacent to the prosthesis assembly (2);
wherein the length of said bolt (16) is such that in the assembled state of the center body member (4) and the plate (3) said bolt (16) passes from said center hole (15) in said plate (3) through the diametral threaded hole (17) of the center body member (4).

When employing the prosthesis, there is, in the first place, removed the vertebra by way of an appropriate surgical technique. Once the cavity for receiving the prosthesis has been prepared, the latter is inserted in the closed position with a minimum height thereof. The prosthesis is held in position with the aid of appropriate clips. Then, it is opened up with a spanner so as to achieve the necessary height for the prosthesis to bite at both the top and bottom ends into the field of the neighbouring vertebrae. The system is locked in place by a plate which is attached by screws laterally to the next above and the next below vertebrae, contributing to stabilization by preventing the antero-posterior translation, lateral rotation and twisting movements.

The prosthesis of the invention offers the advantages that have been described above, apart from others that will be readily gathered from the more detailed description of an embodiment given hereinafter to facilitate an understanding of the features mentioned above, while at the same time disclosing sundry details. The specification is accompanied for this purpose by drawings in which, only as an example and not as a limitation of the scope of the invention, one embodiment of the subject matter is given.

In the drawings,

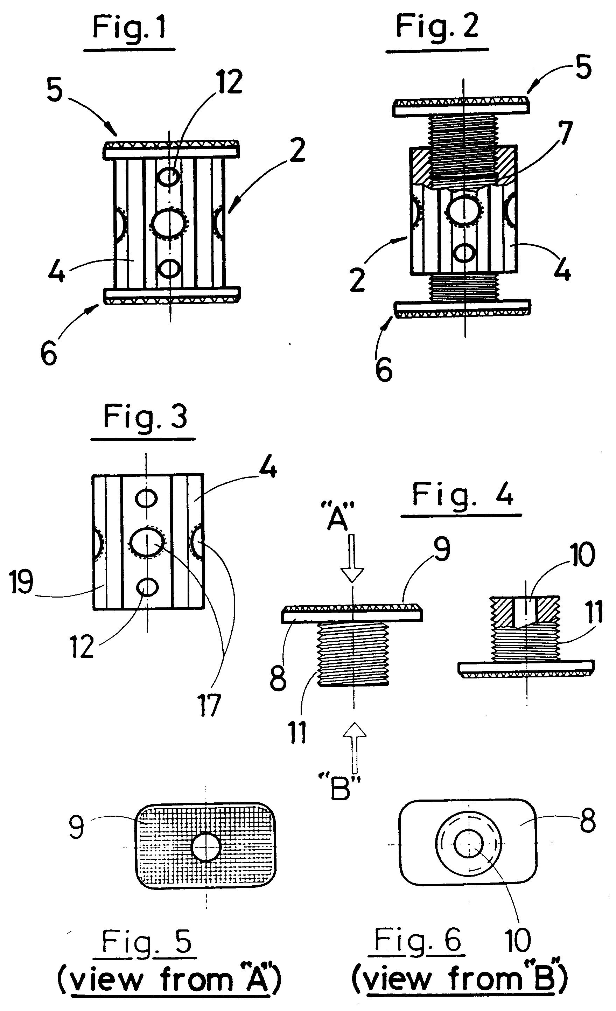

Figure 1 shows the prosthesis assembly in closed position;
Figure 2 shows the prosthesis assembly in the extended state;
Figure 3 is an elevation view of the external hexagonal body forming part of the prosthesis assembly;
Figure 4 shows threaded cylinders forming part of the prosthesis assembly;
Figure 5 is a view of one of the cylinders, seen from "A";
Figure 6 is a view of the said cylinder, seen from "B"; and
Figures 7, 8, 9, 10 and 11 show the different successive steps of a surgical process of placing the prosthesis of the invention between two adjacent vertebrae.

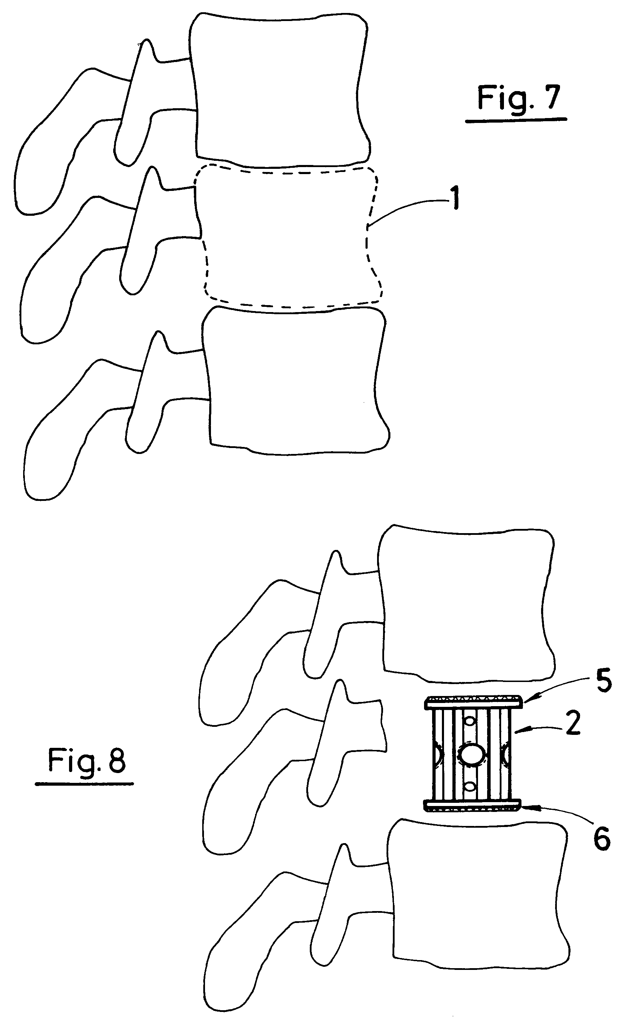

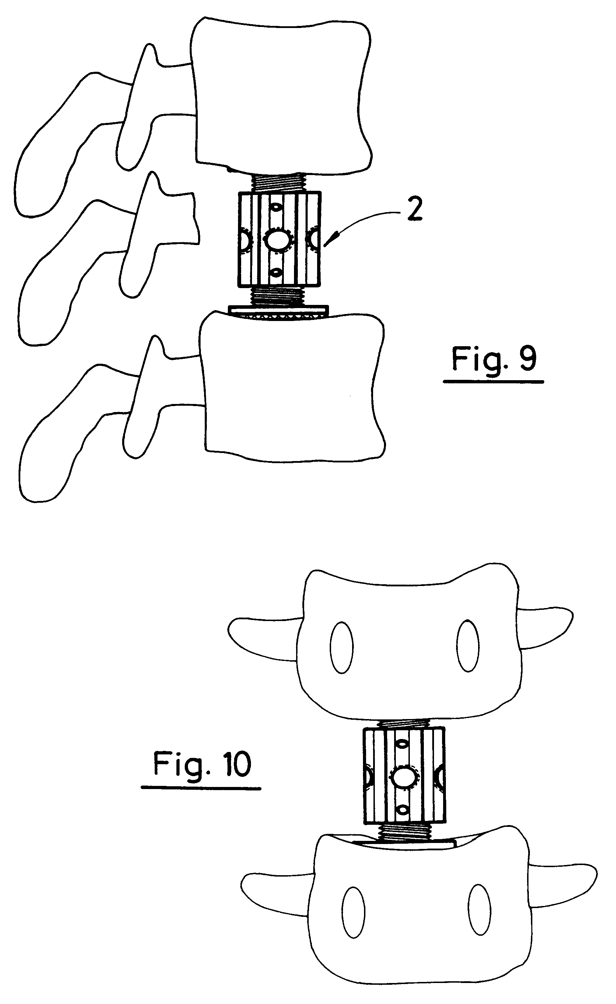

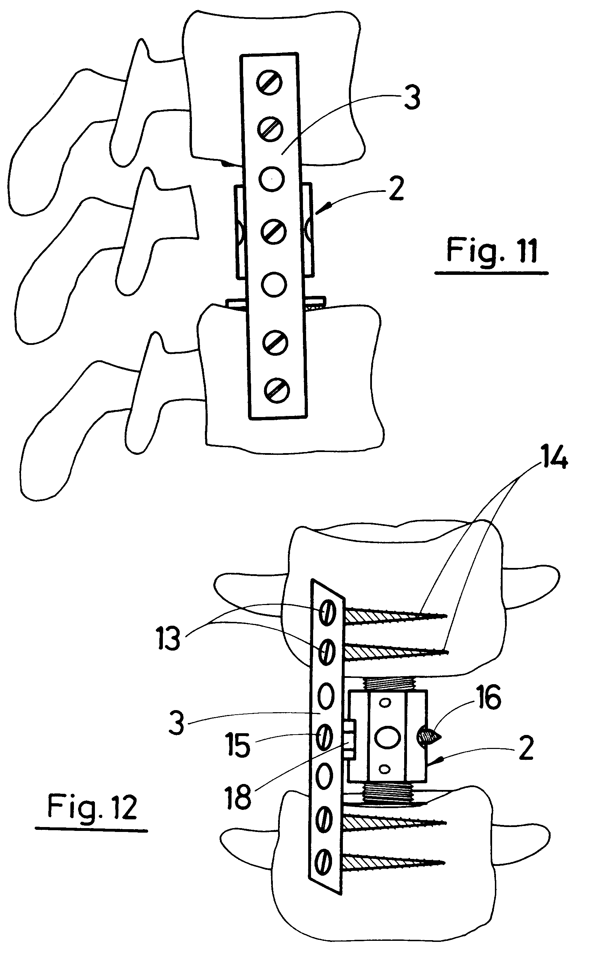

As shown in the drawings, a tumorous vertebra 1 (Figure 7) is removed in a first step. The prosthesis, generally indicated at 2, is inserted in a second step, in the closed state, with the minimum height thereof (Figure 8). The prosthesis 2 is then opened out in a third step with appropriate tools, so that the moving components thereof may move apart and bite into the body of the neighbouring vertebra, as shown in Figure 9, corresponding to a side view, and Figure 10, corresponding to an anteroposterior view. In a fourth step, the system is locked in place by way of a plate 3 which is screwed laterally into the upper and lower vertebrae and compressed, as shown in figures 11 and 12, corresponding to side and antero-posterior views respectively.

The prosthesis 2, used in the manner described above, is made from steel and consists of three portions: a center body member 4 and upper and lower platforms 5 and 6.

The center body member 4 is a hollow tube having an internal thread 7. It is cylindrical on the outside at both ends and has a hexagonal waist in the middle.

Each platform 5 and 6 consists of two parts: a peripheral flat plate 8 having a roughened sheet 9 attached to the outer surface thereof; and a lower hollow cylinder 10, provided with a thread 11 on the outside thereof, which screws into the inside of the center body member 4.

The internal thread 7 of the center body member 4 and the external threads 11 of the cylinders 10 of the respective platforms 5 and 6 are made in such a way that when the center body member 4 is rotated clockwise with a hexagonal spanner, the two cylinders 10 of the platforms 5, 6 simultaneously move axially outwards, thereby extending the height of the assembly. When the center body member 4 is rotated in the opposite direction, the cylinders 10 retract simultaneously into the body member 4, shortening the total height.

The center body member 4 is provided with four small threaded holes 12 at each end. After rotating the center body member in the desired direction, to set the desired height of the prosthesis assembly 2, two small Allen screws are screwed up, one in each of the small holes of the centre body member. In this way, the Allen screws bear against the thread 11 of the cylinders 10 of the platforms 5 and 6 and lock them against further movement.

A plate 3, provided with countersunk self-compression holes 13 for spongiosa screws 14 and a normal center hole 15 for the bolt 16 which passes through the diametral threaded holes 17 of the center body member 4 in combination with a nut 18, is attached to the prosthesis.

The center body member 4 is preferably provided with grooves 19 facilitating the seating of the end of a small chisel with a view to rotating the prosthesis body member on the cylinders 10. The said body member 4 is manipulated with the aid of a spanner, not shown, having wide jaws to take in almost the whole length of the body member 4, facilitating the screwing thereof on the upper and lower cylinders.

## Claims

1. Prosthesis assembly (2) for replacing a vertebra in a spine, said assembly comprising
- a center body member (4) in the form of a hollow tube having an internal thread (7) and diametral threaded holes (17);
- a pair of platform members (5, 6) each having a plate member (8) and a cylinder part (10), said cylinder part (10) having an outer thread (11) to be inserted into one end of said hollow tube of the center body member (4);
- a threaded bolt (16) to be engaged with one of the diametral threaded holes (17) of the center body member (4); and
- a plate (3) including a center hole (15) for receiving the threaded bolt (16) and additional holes (13) for receiving spongiosa screws (14) to be screwed into vertebrae adjacent to the prosthesis assembly (2);
wherein the length of said bolt (16) is such that in the assembled state of the center body member (4) and the plate (3) said bolt (16) passes from said center hole (15) in said plate (3) through the diametral threaded hole (17) of the center body member (4).

2. Prosthesis assembly (2) according to claim 1, wherein said center body member (4) is cylindrical on the outside at both ends and has a hexagonal waist in the middle.

3. Prosthesis assembly (2) according to claim 1 and claim 2, wherein said center body member (4) is provided with grooves (19).

4. Prosthesis assembly (2) according to any of the claims 1 to 3, wherein said diametral hole (17) of said center body member (4) is a threaded hole (17).

5. Prosthesis assembly (2) according to any of the claims 1 to 4, wherein said center body member (4) is provided with threaded holes (12) to receive small screws bearing against said outer threads (11) of said cylinder part(s) (10) of said platform members (5, 6) after their adjustment in said hollow tube of said center body member (4).

6. Prosthesis assembly (2) according to any of the claims 1 to 5, wherein said plate members (8) of said platform members (5, 6) are provided on the outer surface thereof with a roughened sheet (9).

7. Prosthesis assembly (2) according to any of the claims 1 to 6, wherein said plate (3) is in a movement-preventing, releasable engagement with said bolt (16) passing through said diametral hole (17) of the center body member (4).

8. Prosthesis assembly (2) according to claim 7, wherein said movement-preventing, releasable engagement is a fixation by means of a nut (18).

9. Prosthesis assembly (2) according to any of the claims 1 to 8, wherein said plate (3) includes at least four additional holes (13) for receiving spongiosa screws (14) to be screwed into vertebrae adjacent to the prosthesis assembly (2).

## Patentansprüche

1. Prothesen-Anordnung (2) zum Ersetzen eines Wirbels in einer Wirbelsäule, wobei die Anordnung umfaßt:
- ein Zentralkörper-Teil (4) in Form eines hohlen Rohrs mit einem Innengewinde (7) und diametral zueinander angeordneten Löchern (17) mit Gewinde;
- ein Paar von Teilen (5, 6) mit einer Platte, die jeweils einen Platten-Teil (8) und einen Zylinder-Teil (10) aufweisen, wobei der Zylinder-Teil (10) ein Außengewinde (11) aufweist, das in einen Endabschnitt des hohlen Rohrs des Zentralkörper-Teils (4) eingesetzt werden soll;
- eine Schraube (16) mit Gewinde, die mit einem der diametral angeordneten Löcher (17) mit Gewinde des Zentralkörper-Teils (4) in Eingriff gebracht werden soll; und
- eine Platte (3), die ein zentrales Loch (15) zum Aufnehmen der Schraube (16) mit Gewinde und zusätzliche Löcher (13) zum Aufnehmen von Spongiosa-Schrauben (14) einschließt, die in Wirbel eingeschraubt werden sollen, die der Prothesen-Anordnung (2) benachbart sind;
worin die Länge der Schraube (16) derart ist, daß die Schraube (16) im eingebauten Zustand des Zentralkörper-Teils (4) und der Platte (3) von dem zentralen Loch (15) in der Platte (3) durch das diametral angeordnete Loch (17) mit Gewinde des Zentralkörper-Teils (4) reicht.

2. Prothesen-Anordnung (2) nach Anspruch 1, worin das Zentralkörper-Teil (4) auf der Außenseite an beiden Enden zylindrisch ist und einen hexagonalen Bauch in der Mitte aufweist.

3. Prothesen-Anordnung (2) nach Anspruch 1 und Anspruch 2, worin das Zentralkörper-Teil (4) mit Rillen (19) versehen ist.

4. Prothesen-Anordnung (2) nach einem der Ansprüche 1 bis 3, worin das diametral angeordnete Loch (17) des Zentralkörper-Teils (4) ein Loch (17) mit einem Gewinde ist.

5. Prothesen-Anordnung (2) nach einem der Ansprüche 1 bis 4, worin das Zentralkörper-Teil (4) mit mit Gewinde versehenen Löchern (12) zum Aufnehmen kleiner Schrauben versehen ist, die die Außengewinde (11) des/der Zylinder-Teils(e) (10) der Platten-Teile (5, 6) nach deren Justierung in dem hohlen Rohr des Zentralkörper-Teils (4) halten.

6. Prothesen-Anordnung (2) nach einem der Ansprüche 1 bis 5, worin die Platten-Teile (8) der mit Platten versehenen Teile (5, 6) auf ihrer Außenfläche mit einer aufgerauhten Schicht (9) versehen sind.

7. Prothesen-Anordnung (2) nach einem der Ansprüche 1 bis 6, worin die Platte (3) in eine Bewegung verhinderndem, lösbarem Eingriff mit der Schraube (16) ist, die durch das diametral angeordnete Loch (17) des Zentralkörper-Teils (4) reicht.

8. Prothesen-Anordnung (2) nach Anspruch 7, worin der eine Bewegung verhindernde, lösbare Eingriff eine Befestigung mittels einer Mutter (18) ist.

9. Prothesen-Anordnung (2) nach einem der Ansprüche 1 bis 8, worin die Platte (3) wenigstens vier weitere Löcher (13) zum Aufnehmen von Spongiosa-Schrauben (14) einschließt, die in Wirbel eingeschraubt werden sollen, die der Prothesen-Anordnung (2) benachbart sind.

## Revendications

1. Ensemble de prothèse (2) pour remplacer une vertèbre dans une colonne vertébrale, ledit ensemble comprenant
- un élément noyau central (4) en forme de tube creux ayant un filet intérieur (7) et des trous diamétraux filetés (17) ;
- deux éléments de plate-forme (5, 6) ayant chacun un élément de plaque (8) et une partie cylindrique (10), ladite partie cylindrique (10) ayant un filet extérieur (11) devant être introduit dans une partie extrême dudit tube creux de l'élément noyau central (4) ;
- un boulon fileté (16) pour venir en prise avec l'un des trous diamétraux filetés (17) de l'élément noyau central (4) ; et
- une plaque (3) comprenant un trou central (15) pour recevoir le boulon fileté (16) et des trous supplémentaires (13) pour recevoir des vis spongieuses (14) à visser dans des vertèbres adjacentes à l'ensemble de prothèse (2) ;
dans lequel la longueur dudit boulon (16) est telle qu'à l'état assemblé de l'élément noyau central (4) et de la plaque (3), ledit boulon passe dudit trou central (15) de ladite plaque (30) à travers le trou diamétral fileté (17) de l'élément noyau central (4).

2. Ensemble de prothèse (2) selon la revendication 1, dans lequel ledit élément noyau central (4) est cylindrique à l'extérieur aux deux parties extrêmes, et présente au milieu une partie centrale hexagonale.

3. Ensemble de prothèse (2) selon la revendication 1 et la revendication 2, dans lequel l'élément noyau central (4) comporte des rainures (19).

4. Ensemble de prothèse (2) selon l'une quelconque des revendications 1 à 3, dans lequel ledit trou diamétral (17) dudit élément noyau central (4) est un trou fileté (17).

5. Ensemble de prothèse (2) selon l'une quelconque des revendications 1 à 4, dans lequel ledit élément noyau central (4) comporte des trous filetés (12) pour recevoir des petites vis appuyant contre lesdits filets extérieurs (11) desdites parties cylindriques (10) desdits éléments de plate-forme (5, 6) après leur ajustement dans ledit tube creux dudit élément noyau central (4).

6. Ensemble de prothèse (2) selon l'une quelconque des revendications 1 à 5, dans lequel lesdits éléments de plaque (8) desdits éléments de plate-forme (5, 6) comportent sur leur surface extérieure une feuille rugueuse (9).

7. Ensemble de prothèse (2) selon l'une quelconque des revendications 1 à 6, dans lequel ladite plaque (3) est en prise amovible empêchant tout mouvement avec ledit boulon (16) traversant ledit trou diamétral (17) de l'élément noyau central (4).

8. Ensemble de prothèse (2) selon la revendication 7, dans lequel ladite prise amovible empêchant tout mouvement est une fixation au moyen d'un écrou (18).

9. Ensemble de prothèse (2) selon l'une quelconque des revendications 1 à 8, dans lequel ladite plaque (3) comporte au moins quatre trous supplémentaires (13) pour recevoir des vis spongieuses (14) à visser dans des vertèbres adjacentes à l'ensemble de prothèse (2).
